# EUROPEAN PATENT APPLICATION

(11) **EP 3 329 788 A1**
(43) Date of publication of application: **06.06.2018**
(21) Application number: 17204287.1
(22) Date of filing: 29.11.2017
(51) Int. Cl.: A23L 33/175, A61K 31/198, A23L 7/126, A23L 33/00, A61P 3/02

(54) **FOOD FOR PROPHYLAXIS OR IMPROVEMENT OF MALNUTRITION**

(30) Priority: 02.12.2016 FR 1661851
(71) Applicant: AJINOMOTO CO., INC., Chuo-ku Tokyo 104-8315 (JP)
(72) Inventor: Murakami, Hitoshi, Tokyo, 104-8315 (JP); Honda, Masashi, Tokyo, 104-8315 (JP)
(74) Representative: Strehl Schübel-Hopf & Partner

(57) **Abstract**

The present invention aims to provide a food containing, as a main ingredient, a foodstuff produced in developing regions with many malnourished patients, specifically at least one kind of grain selected from the group consisting of soybean, maize and sorghum, which contains sufficient protein/amino acid nutrients, is easy to take even without water, and provides a high improving effect on malnutrition (especially severe acute malnutrition).

The present invention relates to a food containing at least one grain selected from the group consisting of soybean, maize and sorghum, lysine or a salt thereof, glutamine or a salt thereof, and methionine or a salt thereof.

## Description

### TECHNICAL FIELD OF THE INVENTION

The present invention relates to a food comprising at least one kind of grain selected from the group consisting of soybean, maize and sorghum as a main ingredient, which provides sufficient protein/amino acid nutrition, is easily ingested without water, and shows a high improving effect on malnutrition.

### BACKGROUND OF THE INVENTION

In the world, about one billion people are suffering from starvation and malnutrition, and not less than 3 million infants of less than 5 years old die annually due to malnutrition.

In recent years, Ready-to-Use Therapeutic Foods (RUTFs) represented by "Plumpy'nut" (trade name) is used for the treatment of severe acutely malnourished children (WO 2005/096837 A2 etc.). RUTFs are characterized in that (1) they have high nutritional value, (2) they do not require cooking for ingestion, (3) they are easily ingested even without water, (4) they are individually sealed with aluminum packaging etc., hygienic and easy to distribute, (5) they can be preserved for a long period even at high temperature, and the like, and affording significant results in nutritional treatment and nutritional support of malnourished children and the like.

While the aforementioned "Plumpy'nut" contains dried skim milk as one of the main ingredients, milk and other animal-derived materials are very expensive and often imported in the developing regions where many malnourished patients live. In those areas, therefore, such foods are considered to be unsuitable for eating habits and food culture in the areas, since the production of such foods is limited, they are mostly imported from developed countries, and people in those areas have fewer experience of eating such foods compared to cereals. For example, in Africa and the like, there are many children with lactose intolerance, and if such children ingest food containing a large amount of milk, they are considered to develop diarrhea and have deteriorated nutritional condition.

The aforementioned "Plumpy'nut" contains a large amount of peanut. Peanuts are more frequently contaminated with aflatoxin than other plant-derived materials. Aflatoxin is known to be highly carcinogenic and to inhibit nutrient absorption. Therefore, using peanut as a raw material for nutritional food or nutritional treatment food is highly risky.

If RUTFs can be produced from ingredients produced in developing regions with many malnourished patients as starting materials, RUTFs can be provided at a low cost in a timely manner when needed even in such areas, and further, stimulation of the area's economy can also be expected.

On the other hand, plant-derived materials mainly produced in developing regions contain insufficient amount of protein nutrition as compared to animal-derived materials, and improvement thereof is important for providing food with high nutritional value.

### SUMMARY OF THE INVENTION

The present invention has been made in view of the aforementioned situation, and aims to provide a food containing, as a main ingredient, a foodstuff produced in developing regions with many malnourished patients, specifically at least one kind of grain selected from the group consisting of soybean, maize and sorghum, which contains sufficient protein/amino acid nutrients, is easy to take even without water, and provides a high improving effect on malnutrition (especially severe acute malnutrition).

The present inventors have conducted intensive studies in an attempt to solve the aforementioned problem and found that malnutrition can be effectively improved by combining at least one kind of grain selected from the group consisting of soybean, maize and sorghum with 3 to 5 particular kinds of amino acids, and conducted further studies based on such finding and completed the present invention.

Accordingly, the present invention provides the following.
[1] A food comprising at least one grain selected from the group consisting of soybean, maize and sorghum, lysine or a salt thereof, glutamine or a salt thereof, and methionine or a salt thereof, wherein
   a content of the lysine or a salt thereof is 0.01 - 3 wt% relative to the food,
   a content of the glutamine or a salt thereof is 0.01 - 3 wt% relative to the food, and
   a content of the methionine or a salt thereof is 0.001 - 3 wt% relative to the food.
[2] The food of [1], wherein a content of the aforementioned grain is 20 - 60 wt% relative to the food,
   the content of the lysine or a salt thereof is 0.1 - 0.5 wt% relative to the food,
   the content of the glutamine or a salt thereof is 0.1 - 0.8 wt% relative to the food, and
   the content of the methionine or a salt thereof is 0.01 - 0.3 wt% relative to the food.
[3] The food of [1] or [2], wherein the aforementioned grain includes soybean, maize and sorghum.
[4] The food of [3], wherein
   a content of the soybean is 15 - 50 wt% relative to the food,
   a content of the maize is 1 - 10 wt% relative to the food, and
   a content of the sorghum is 0.5 - 5 wt% relative to the food.
[5] The food of any one of [1] - [4], further comprising at least one selected from proline or a salt thereof, and glycine or a salt thereof.
[6] The food of any one of [1] - [5], further comprising oil.
[7] The food of any one of [1] - [6], which does not contain milk or contains milk at not more than 20 wt% relative to the food.
[8] The food of any one of [1] - [7], which is substantially free of peanut.
[9] The food of any one of [1] - [8], which is for the prophylaxis or improvement of malnutrition.
[10] The food of [9], wherein the malnutrition is severe acute malnutrition.
[11] The food of any one of [1] - [10], which is a Ready-to-Use Therapeutic Food.
[12] Use of the food of any one of [1] - [11], which is for the prophylaxis or improvement of malnutrition.
[13] The use of [12], wherein the malnutrition is severe acute malnutrition.

The food of the present invention can be provided at a low cost in developing regions with many malnourished patients and is suitable for the dietary habit and dietary culture in the areas, since it is mainly composed of foodstuffs (soybean, maize and sorghum) produced in the area, is easy to ingest even without water, and provides a malnutrition improving effect equal to or higher than that of conventional Ready-to-Use Therapeutic Foods. In addition, the food of the present invention can provide an effect equal to or higher than that provided when it contains an animal-derived material such as milk. Therefore, the food of the present invention is highly useful for the nutritional treatment and nutritional support of malnourished patients in developing regions.

### BRIEF DESCRIPTION OF THE DRAWINGS

Fig. 1 is a graph showing changes in the body length from the start of ingestion of a test food (CMS, CMC and CMS+AA) in an animal test until 3 weeks later. Different letters indicate significant differences determined by Tukey-Kramer multiple comparison test after ANOVA.
Fig. 2 is a graph showing changes in the serum albumin concentration from the start of ingestion of a test food (CMS, CMC and CMS+AA) in an animal test until 3 weeks later. Different letters indicate significant differences determined by Tukey-Kramer multiple comparison test after ANOVA.
Fig. 3 is a graph showing the recovery rate after ingestion of a test food in a human test.
Fig. 4 is a graph showing the amount of change in blood hemoglobin concentration before the start of the test and after the completion of the test in a human test.

### DESCRIPTION OF EMBODIMENTS

The food of the present invention is mainly characterized in that it contains at least one kind of grain selected from the group consisting of soybean, maize and sorghum, lysine or a salt thereof, glutamine or a salt thereof, and methionine or a salt thereof.

The food of the present invention preferably contains at least one kind of grain selected from the group consisting of soybean, maize and sorghum, lysine or a salt thereof, glutamine or a salt thereof, and methionine or a salt thereof, and further, at least one selected from proline or a salt thereof, and glycine or a salt thereof. The food of the present invention further containing at least one selected from proline or a salt thereof, and glycine or a salt thereof is expected to promote synthesis of nucleic acid and/or glutathione.

The food of the present invention particularly preferably contains at least one kind of grain selected from the group consisting of soybean, maize and sorghum, lysine or a salt thereof, glutamine or a salt thereof, methionine or a salt thereof, proline or a salt thereof, and glycine or a salt thereof.

Soybean used in the present invention is a seed (including dried product) of a plant of Order: Fabales, Family: Fabaceae, Genus: Glycine. Defatted soybean obtained by extracting oil from the seed or a processed product thereof (e.g., soybean protein etc.) may also be used.

When the food of the present invention contains soybean, the content of soybean in the food of the present invention is generally 15 - 50 wt% relative to the food of the present invention and, from the aspects of nutrition composition, property and easiness of eating, preferably 17 - 45 wt%, more preferably 23 - 38 wt%. In the present invention, the weight of soybean, maize and sorghum is a dry weight unless particularly specified.

The maize used in the present invention is a fruit (including dried product) of a plant of Order: Poales, Family: Poaceae, Genus: Zea.

When the food of the present invention contains maize, the content of maize in the food of the present invention is generally 1 - 10 wt% relative to the food of the present invention and, from the aspects of nutrition composition, property and easiness of eating, preferably 5 - 7 wt%, more preferably 5.2 - 6.8 wt%.

When the food of the present invention contains soybean and maize, the weight ratio of the content of soybean and the content of maize (soybean:maize) in the food of the present invention is preferably 1:0.05 - 0.7, more preferably 1:0.1 - 0.3, from the aspects of nutrition composition, property and easiness of eating.

Sorghum used in the present invention is a fruit (including dried product) of a plant of Order: Poales, Family: Poaceae, Genus: Sorghum.

When the food of the present invention contains sorghum, the content of sorghum in the food of the present invention is generally 0.5 - 5 wt% relative to the food of the present invention and, from the aspects of nutrition composition, property and easiness of eating, preferably 3 - 4 wt%, more preferably 3.2 - 3.7 wt%.

When the food of the present invention contains soybean and sorghum, the weight ratio of the content of soybean and the content of sorghum (soybean:sorghum) in the food of the present invention is preferably 1:0.01 - 0.3, more preferably 1:0.08 - 0.2, from the aspects of nutrition composition, property and easiness of eating.

When the food of the present invention contains maize and sorghum, the weight ratio of the content of maize and the content of sorghum (maize:sorghum) in the food of the present invention is preferably 1:0.1 - 1, more preferably 1:0.4 - 0.8, from the aspects of nutrition composition, property and easiness of eating.

The content of at least one kind of grain selected from the group consisting of soybean, maize and sorghum in the food of the present invention is generally 20 - 60 wt% relative to the food of the present invention and, from the aspects of nutrition composition, property and easiness of eating, preferably 25 - 56 wt%, more preferably 33 - 48 wt%. The content of the grain when the food of the present invention contains only one selected from the group consisting of soybean, maize and sorghum is the content thereof, and when the food of the present invention contains 2 or 3 selected from the group consisting of soybean, maize and sorghum, the content is the total of said 2 or 3.

The food of the present invention preferably contains all of soybean, maize and sorghum as the grain.

The grain (soybean, maize, sorghum) to be used as the starting material of the food of the present invention is preferably pulverized and used in the form of granule, powder or the like, from the aspects of properties and easiness of eating.

Lysine, glutamine, methionine, proline, glycine and salts thereof to be used in the present invention can be obtained by extraction and purification from naturally occurring animals and plants, or obtained by chemical synthesis method, fermentation method, enzymatic method or gene recombination method. Lysine, glutamine, methionine, proline, glycine and salts thereof to be used in the present invention may be in any of L-, D- and DL-forms, preferably L-form.

The salts of lysine, glutamine, methionine, proline and glycine to be used in the present invention are preferably pharmacologically acceptable salts and, for example, salts with inorganic acids, salts with organic acids, salts with inorganic bases, salts with organic bases and the like can be mentioned.

Examples of the salts with inorganic acids include salts with hydrohalic acid (hydrochloric acid, hydrobromic acid, hydroiodic acid etc.), sulfuric acid, nitric acid, phosphoric acid or the like.

Examples of the salts with organic acids include salts with formic acid, acetic acid, propionic acid, oxalic acid, succinic acid, maleic acid, fumaric acid, citric acid or the like.

Examples of the salts with inorganic bases include salts with alkali metals such as sodium, potassium, lithium and the like, salts with alkaline earth metals such as calcium, magnesium and the like, salt with ammonium and the like.

Examples of the salts with organic bases include salts with ethylenediamine, propylenediamine, ethanolamine, monoalkylethanolamine, dialkylethanolamine, diethanolamine, triethanolamine or the like.

The form of lysine, glutamine, methionine, proline and glycine to be used in the present invention is not particularly limited and may be any form such as a free form, a peptide form, a protein form, a salt form or a mixture thereof. From the aspects of stability and use effects of the products, a free form is preferable. Lysine, glutamine, methionine, proline, glycine and salts thereof to be used in the present invention are preferably purified, from the aspects of stability and use effects of the products.

The content of lysine or a salt thereof in the food of the present invention is generally 0.01 - 3 wt% relative to the food of the present invention and, from the aspect of protein/amino acid nutrition, preferably 0.1 - 0.5 wt%, more preferably 0.25 - 0.42 wt%.

The calculation of the amount of amino acid salt in the present invention is based on a free form.

The weight ratio of the content of at least one kind of grain selected from the group consisting of soybean, maize and sorghum and the content of lysine or a salt thereof (grain:lysine or a salt thereof) in the food of the present invention is preferably 1:0.0015 - 0.025, more preferably 1:0.005 - 0.015, from the aspect of protein/amino acid nutrition.

The content of glutamine or a salt thereof in the food of the present invention is generally 0.01 - 3 wt% relative to the food of the present invention and, from the aspect of protein/amino acid nutrition, preferably 0.1 - 0.8 wt%, more preferably 0.3 - 0.6 wt%.

The weight ratio of the content of at least one kind of grain selected from the group consisting of soybean, maize and sorghum and the content of glutamine or a salt thereof (grain:glutamine or a salt thereof) in the food of the present invention is preferably 1:0.0015 - 0.04, more preferably 1:0.006 - 0.017, from the aspect of protein/amino acid nutrition.

The weight ratio of the content of lysine or a salt thereof and the content of glutamine or a salt thereof (lysine or a salt thereof:glutamine or a salt thereof) is preferably 1:0.2 - 8, more preferably 1:0.8 - 2, from the aspect of protein/amino acid nutrition.

The content of methionine or a salt thereof in the food of the present invention is generally 0.001 - 3 wt% relative to the food of the present invention and, from the aspect of protein/amino acid nutrition, preferably 0.01 - 0.3 wt%, more preferably 0.05 - 0.2 wt%.

The weight ratio of the content of at least one kind of grain selected from the group consisting of soybean, maize and sorghum and the content of methionine or a salt thereof (grain:methionine or a salt thereof) in the food of the present invention is preferably 1:0.0002 - 0.015, more preferably 1:0.001 - 0.0065, from the aspect of protein/amino acid nutrition.

The weight ratio of the content of lysine or a salt thereof and the content of methionine or a salt thereof (lysine or a salt thereof:methionine or a salt thereof) is preferably 1:0.02 - 3, more preferably 1:0.1 - 0.7, from the aspect of protein/amino acid nutrition.

When the food of the present invention contains proline or a salt thereof, the content of proline or a salt thereof in the food of the present invention is generally 0.01 - 3 wt% relative to the food of the present invention and, from the aspect of protein/amino acid nutrition, preferably 0.2 - 0.8 wt%, more preferably 0.3 - 0.8 wt%.

When the food of the present invention contains proline or a salt thereof, the weight ratio of the content of at least one kind of grain selected from the group consisting of soybean, maize and sorghum and the content of proline or a salt thereof (grain:proline or a salt thereof) is preferably 1:0.003 - 0.04, more preferably 1:0.006 - 0.025, from the aspect of protein/amino acid nutrition.

When the food of the present invention contains proline or a salt thereof, the weight ratio of the content of lysine or a salt thereof and the content of proline or a salt thereof (lysine or a salt thereof:proline or a salt thereof) in the food of the present invention is preferably 1:0.4 - 8, more preferably 1:0.75 - 2.7, from the aspect of protein/amino acid nutrition.

When the food of the present invention contains glycine or a salt thereof, the content of glycine or a salt thereof in the food of the present invention is generally 0.01 - 3 wt% relative to the food of the present invention and, from the aspect of protein/amino acid nutrition, preferably 0.2 - 0.5 wt%, more preferably 0.25 - 0.42 wt%.

When the food of the present invention contains glycine or a salt thereof, the weight ratio of the content of at least one kind of grain selected from the group consisting of soybean, maize and sorghum and the content of glycine or a salt thereof (grain:glycine or a salt thereof) is preferably 1:0.003 - 0.025, more preferably 1:0.005 - 0.013, from the aspect of protein/amino acid nutrition.

When the food of the present invention contains glycine or a salt thereof, the weight ratio of the content of lysine or a salt thereof and the content of glycine or a salt thereof (lysine or a salt thereof:glycine or a salt thereof) in the food of the present invention is preferably 1:0.4 - 5, more preferably 1:0.6 - 1.4, from the aspect of protein/amino acid nutrition.

The food of the present invention preferably further contains oil in addition to at least one grain selected from the group consisting of soybean, maize and sorghum, and the above-mentioned 3 to 5 kinds of amino acids. Oil can be one of the energy sources in the food of the present invention, and the food of the present invention containing oil can take a form permitting easy ingestion even without water.

While the oil usable for the food of the present invention is not particularly limited as long as it is edible, palm oil, palm kernel oil, palmolein, palm stearin, olive oil, soybean oil, rape seed oil, almond oil, safflower oil, sunflower oil, corn oil, avocado oil, cottonseed oil and the like can be recited as examples.

When the food of the present invention contains oil, the content of oil in the food of the present invention is generally 20 - 40 wt% relative to the food of the present invention and, from the aspect of nutritional requirements, preferably 22 - 35 wt%.

The food of the present invention may further contain, in addition to the above-mentioned starting materials, other starting material. While such other starting material is not particularly limited as long as it does not impair the object of the present invention, for example, sugar, trace nutrient (vitamin, mineral) and the like can be mentioned. The content of such other starting material is generally not more than 3.5 wt%, preferably not more than 3.0 wt%, relative to the food of the present invention.

It is preferable that the food of the present invention does not contain milk from the economical point of view, so that it can be provided at a low cost in developing regions. In the present invention, "milk" is a concept including a liquid secreted from the mammary gland of a female mammal (e.g., cow, goat, etc.) and a product group obtained by processing the liquid. Specific examples of the milk include raw milk, cows milk, raw goat milk, sterilized goat milk, composition-adjusted milk, low-fat milk, fat-free milk, cream, butter, butter oil, cheese, concentrated whey, concentrated milk, concentrated skim milk, evaporated milk, evaporated skim milk, sweetened condensed milk, sweetened condensed skim milk, powdered whole milk, dried skim milk, cream powder, whey powder, protein concentrated whey powder, powdered buttermilk, sweetened powdered milk, formulated powdered milk, fermented milk and the like.

When the food of the present invention contains milk, the content thereof is, from the economical point of view, preferably not more than 20 wt%, more preferably not more than 15 wt%, particularly preferably not more than 10 wt%, relative to the food of the present invention.

It is preferable that the food of the present invention does not contain milk or contains milk at not more than 20 wt% (more preferably not more than 15 wt%) relative to the food of the present invention.

The food of the present invention is preferably substantially free of peanut. Being "substantially free of" peanut here means either (a) completely free of peanut, or (b) containing peanut at not more than 1 wt% (preferably not more than 0.5 wt%) relative to the food.

The production method of the food of the present invention is not particularly limited, and can be produced by a method known per se. For example, the food of the present invention can be produced by pulverizing at least one grain selected from the group consisting of soybean, maize and sorghum, lysine or a salt thereof, glutamine or a salt thereof, and methionine or a salt thereof each into a powder, mixing them, adding oil and kneading the mixture and the like.

While the form of the food of the present invention is not particularly limited, a solid or semi-solid form (including paste form and slurry form) is preferable. The form of the food of the present invention is particularly preferably a paste form because it is easy to ingest.

When the food of the present invention is a semi-solid, the hardness of the food of the present invention is preferably not more than 100,000 g.sec, more preferably not more than 40,000 g.sec, particularly preferably not more than 30,000 g. sec, from the aspect of usability.

In the present invention, the hardness of the food is measured with a texture analyzer, and specifically means a repulsive force when a rectangular plunger of 3.8 cm × 5.0 cm is pushed into the food for 10 mm at 1 mm/sec. The hardness of the food of the present invention can be appropriately controlled by, for example, adjusting the amount of air taken into the food.

The calorific value per 100 g of the food of the present invention is preferably 520 to 550 kcal.

The water activity (Aw) of the food of the present invention is generally not more than 0.65.

In the present invention, Aw of the food is determined from an adsorption isotherm.

The water content of the food of the present invention is generally not more than 8 wt%, preferably not more than 6 wt%.

In the present invention, the water content of the food is measured by an atmospheric heating drying method.

The food of the present invention has a fat content of generally 10 - 50 wt%, preferably 20 - 40 wt%.

In the present invention, the fat content of the food is measured by an acidolysis method.

The food of the present invention can be provided as a packaged product. The packaging material that can be used for packaging the food of the present invention is not particularly limited as long as it is usually used for packaging foods, and examples thereof include aluminum foil and the like. The weight per packaged food of the present invention (excluding the weight of packaging material) is usually 90 to 98 g, preferably 90 - 95 g.

From the viewpoint of convenience, the food of the present invention is preferably a food that can be directly ingested without cooking (i.e., Ready-to-Use Food).

The food of the present invention can be ingested by a mammal (e.g., human, bovine, horse, dog, cat, monkey, mouse, rat etc.) to prevent or improve malnutrition. "Malnutrition" in the context of the present invention refers to an abnormal state of the body caused by a shortage of food intake or an imbalance between demand and supply of one or more nutrients, and is a concept including both acute malnutrition and chronic malnutrition.

As a specific criterion for determining malnutrition, for example, in a human, when the Weight for Height Z score (WHZ), which is an index of the body weight per height, is not more than -2 and/or Middle Upper Arm Circumstance (MUAC) is not more than 125 mm, the human is judged as malnourished.

In the present invention, "prevention" of malnutrition refers to preventing or delaying malnutrition, and "improvement" of malnutrition means to get out of malnutrition, treat or alleviate the symptoms of malnutrition partly or entirely, or prevent or delay the exacerbation of the symptoms of malnutrition.

Specific examples of the symptoms of malnutrition include anemia and the like. In chronic malnutrition, for example, symptoms such as anemia and the like can be confirmed chronically.

The food of the present invention can be preferably used for the prophylaxis or improvement of severe acute malnutrition among malnutrition.

As a specific criterion for determining severe acute malnutrition, for example, in a human, when a Weight for Heart Z score (WHZ), which is an index of body weight per height, is not more than -3 and/or a Middle Upper Arm Circumstance (MUAC) is not more than 115 mm, the human is judged to have severe acute malnutrition.

The amount of the food of the present invention to be ingested by an animal varies depending on the kind, age, nutritional condition and the like of the animal. For example, when ingested by an infant from 6 months to 5 years old with malnutrition, it is usually not less than 200 kcal/kg/day, which amount is preferably ingested in 1 to 5 divided portions (preferably 2 to 4 portions) per day.

The food of the present invention can be preferably used as a Ready-to-Use Therapeutic Food.

### Examples

The present invention is explained in more detail in the following by referring to Examples; however, the present invention is not limited by the following Examples.

### (animal test)

3-Week-old male Wistar rats were preliminarily bred on a cornmeal food for 10 days, divided into 3 groups (n=8), and each group ingested any of the test foods (CMS, CMC and CMS+AA) (same calorie) described in Table 1 below for 3 weeks.

**[Table 1]**

| | CMS (parts by weight) | CMC (parts by weight) | CMS+AA (parts by weight) |
|---|---|---|---|
| cornmeal | 100.00 | 100.00 | 100.00 |
| soy protein | 6.06 | - | 6.06 |
| casein | - | 6.44 | - |
| soy bean oil | 3.00 | 3.00 | 3.00 |
| AIN93G mineral mix | 3.00 | 3.00 | 3.00 |
| AIN93 vitamin mix | 0.90 | 0.90 | 0.90 |
| choline bitartrate | 0.25 | 0.25 | 0.25 |
| t-butylhydroquinone | 0.0014 | 0.0014 | 0.0014 |
| lysine | - | - | 0.300 |
| glutamine | - | - | 0.200 |
| methionine | - | - | 0.100 |
| proline | - | - | 0.250 |
| glycine | - | - | 0.150 |

The mice were fasted for 4 hr and the body length (length from the neck to the root of the tail (Neck-hip length)) was measured under anesthesia before the start of ingestion of the test food and 1, 2 and 3 weeks after the start of ingestion of the test food. Blood was taken from the tail vein before the start of ingestion of the test food and three weeks after the start of ingestion of the test food, and the concentration of blood albumin, which is one of indicators showing the protein nutritional status in the body, was measured.

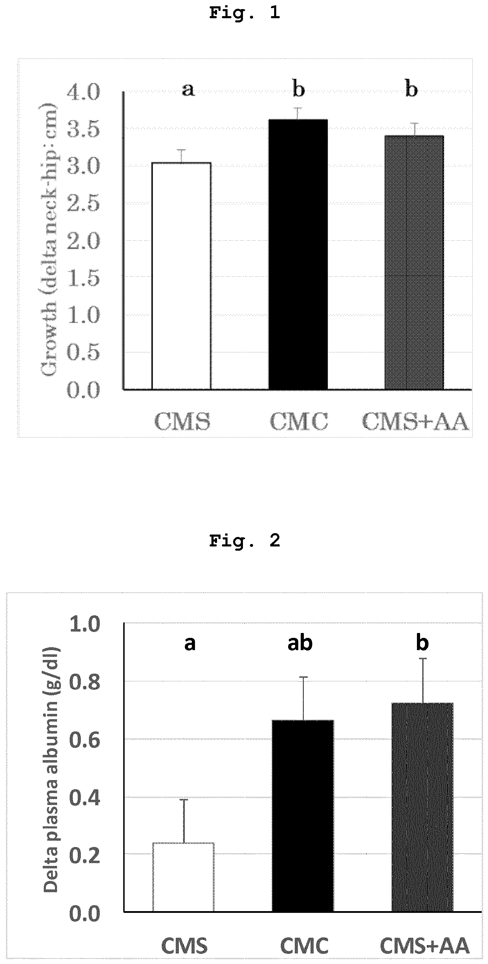

Changes in the body length from the start of ingestion of the test food until three weeks later are shown in Fig. 1. Significant improvement of growth was observed in the group that ingested CMC (CMC group) and the group that ingested CMS+AA (CMS+AA group) as compared to the group that ingested CMS (CMS group). Growth improvement almost similar to that of the CMC group was observed in the CMS+AA group.

Fig. 2 shows changes in the blood albumin concentration from the start of ingestion of the test food until 3 weeks later. Compared to the CMS group, the CMC group showed a tendency toward improvement of the protein nutritional status, and the CMS+AA group showed a significant improvement in the protein nutritional status.

From the above results, it was found that ingestion of lysine, glutamine, methionine, proline and glycine in addition to soybean and maize affords a nutrition improvement effect equal to or higher than that obtained when casein as a milk protein was ingested, which shows a possibility that the combination of grain and amino acid may replace the milk protein.

### (Human study)

The human study was a three arms statistician blinded, randomized and controlled, parallel group, efficacy non-inferiority trial.

### <Subject>

The children aged 6 to 59 months with SAM (severe acute malnutrition) were recruited in this study. The number of SAM children allocated for P-RUTF (milk-peanuts type RUTF as control) treatment group was 474 (number of children under 24 months of age: 293, number of children over 24 months of age: 181). The number of SAM children allocated for MSMS (low milk content Soya-Maize-Sorghum RUTF) treatment group was 441 (number of children under 24 months of age: 255, number of children over 24 months of age: 186). The number of SAM children allocated for FSMS (milk free Soya-Maize-Sorghum RUTF) treatment group was 486 (number of children under 24 months of age: 288, number of children over 24 months of age: 198).

The study was carried out in the Central Region of Malawi at 21 different health centres distributed in 3 districts. The enrolled children attended the each site daily from 9 a.m. to 3 p.m. and were treated with any one of the three types of RUTFs (MSMS, FSMS and P-RUTF).

### <Treatment>

The children took any one of the three RUTFs (MSMS, FSMS and P-RUTF) by fee-feeding (200kcal/kg/day at least). The three types of RUTFs were respectively ingested in the same manner as in the national guidelines for the treatment of SAM, except that the day-care approach, which was called CMAM (Community based Management of Acute Malnutrition), was used. The ingestions of RUTFs were continued around 2 months until achieving discharge criteria.

### <study RUTFs>

In this study, three types of RUTFs were used. One of the used RUTFs was standard milk-peanut type RUTF, which was called P-RUTF, as control. Other two of the used RUTFs were milk free Soya-Maize-Sorghum RUTF (FSMS) and low milk content Soya-Maize-Sorghum RUTF (MSMS). The composition of each of the three RUTFs is shown in following Table 2 or 3.

**[Table 2]**

| | FSMS (wt %) | MSMS (wt %) |
|---|---|---|
| soy flour | 28.50 | 9.42 |
| maize | 5.50 | 6.60 |
| sorghum | 3.50 | 3.40 |
| soy flour defatted | 8.50 | 14.08 |
| dried skim milk | - | 9.30 |
| palm oil | 23.50 | 30.46 |
| palm stearin | 4.00 | 2.00 |
| sugar | 22.50 | 20.50 |
| L-lysine/HCl | 0.38 | 0.32 |
| L-glutamine | 0.50 | 0.40 |
| L-metionine | 0.12 | 0.08 |
| glycine | - | 0.08 |
| L-proline | 0.50 | 0.37 |
| micronutrients | 2.50 | 3.00 |

**[Table 3]**

| | P-RUTF (wt%) |
|---|---|
| dried skim milk | 25.00 |
| peanut paste | 23.00 |
| canola oil | 5.00 |
| palm oil | 11.00 |
| palm stearin | 4.00 |
| myverol | 1.00 |
| sugar | 29.40 |
| micronutrients | 1. 60 |
| total | 100.00 |

The water contents and fat contents of FSMS and MSMS are shown in Table 4 below.

**[Table 4]**

| | FSMS | MSMS |
|---|---|---|
| water content (wt%) | 2.2 | 2.9 |
| fat content (wt%) | 34.2 | 36.0 |

The water contents of FSMS and MSMS were measured by an atmospheric heating drying method.

The fat contents of FSMS and MSMS were measured by an acidolysis method.

### <Admission and discharge criteria>

Admission and discharge criteria in this study were following (Table 5).

**[Table 5]**

| | |
|---|---|
| Admission | MUAC < 115 mm Bilateral pitting oedema grade + or ++ WFH Z-score <- 3 (WHO 2006 curves) |
| Discharge | Absence of bilateral pitting oedema for 14 successive days |
| | MUAC>=125 mm for 14 successive days |
| | Absence of acute life threatening infection |

### <Measurement>

Anthropometric parameters (weight and MUAC; Middle Upper Arm Circumstance) were measured each day.

The blood hemoglobin concentration at the start of the test and completion of the test were measured by Haemocue®.

### <calculation of recovery rate>

Subjects who had MUAC (Middle Upper Arm Circumstance) of not less than 115 mm for 2 weeks or more were judged to have recovered from malnutrition, and the ratio of the number of subjects who recovered from malnutrition relative to the total number of subjects was calculated (recovery rate).

### <calculation of amount of change of blood hemoglobin concentration>

Blood hemoglobin concentration at the time of the start of the test was subtracted from the blood hemoglobin concentration at the completion of the test, and the amount of change of the blood hemoglobin concentration was calculated.

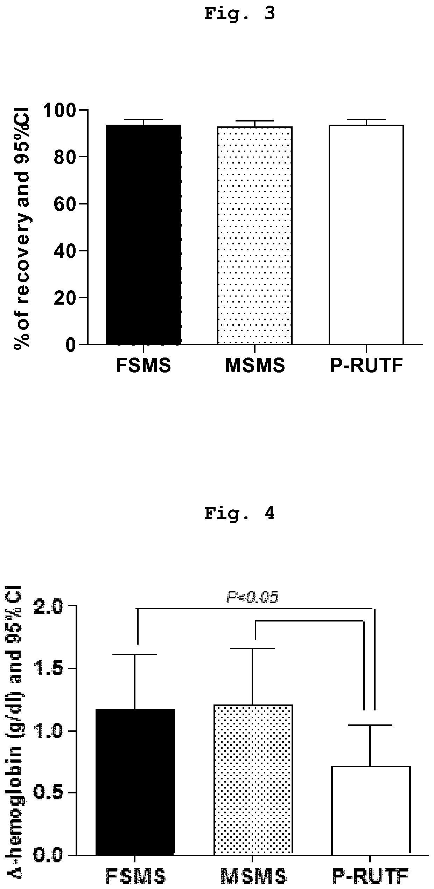

The results are shown in Fig. 3 (recovery rate) and Fig. 4 (amount of change of blood hemoglobin concentration). The test subjects who dropped out from the test were excluded from the results.

From the results shown in Fig. 3, it was confirmed that the food of the present invention (FSMS and MSMS) has a malnutrition improvement effect which were not inferior to that of P-RUTF by Per Protocol analysis.

The results shown in Fig. 4 were statistically processed using a paired t-test, and the food of the present invention (FSMS and MSMS) showed a significant difference of p<0.05 as compared to P-RUTF. Therefore, it was confirmed that the food (FSMS and MSMS) of the present invention is superior in the recovery effect from anemia, as compared to P-RUTF.

### Production Example 1

1) soybean 28.50 parts by weight
2) maize 5.50 parts by weight
3) sorghum 3.50 parts by weight
4) defatted soybean 8.50 parts by weight
5) palm oil 23.50 parts by weight
6) palm stearin 4.00 parts by weight
7) sugar 22.50 parts by weight
8) lysine hydrochloride 0.380 parts by weight
9) glutamine 0.500 parts by weight
10) L-methionine 0.120 parts by weight
11) L-proline 0.500 parts by weight
12) trace nutrient 2.50 parts by weight
   total 100 parts by weight
   1) - 4) were mixed, 7) was added and they were preliminarily mixed. 5) and 6) were mixed, a half amount of a mixture of 1) - 4) and 7) was added and mixed therewith, 8) - 12) were added and they were mixed, and finally, the rest of the mixture of 1) - 4) and 7) was added and mixed.

### Production Example 2

1) soybean 9.42 parts by weight
2) maize 6.60 parts by weight
3) sorghum 3.40 parts by weight
4) defatted soybean 14.08 parts by weight
5) powdered skim milk 9.30 parts by weight
6) palm oil 30.46 parts by weight
7) palm stearin 2.00 parts by weight
8) sugar 20.50 parts by weight
9) lysine hydrochloride 0.320 parts by weight
10) glutamine 0.400 parts by weight
11) L-methionine 0.075 parts by weight
12) glycine 0.075 parts by weight
13) L-proline 0.370 parts by weight
14) trace nutrient 3.00 parts by weight
   total 100 parts by weight
   1) - 4) were mixed, 8) was added and they were preliminarily mixed. 6) and 7) were mixed, a half amount of a mixture of 1) - 4) and 8) was added and mixed therewith, 5) and 9) - 14) were added and they were mixed, and finally, the rest of the mixture of 1) - 4) and 7) was added and mixed.

The food of the present invention can be provided at a low cost in developing regions with many malnourished patients, since it is mainly composed of foodstuffs (soybean, maize and sorghum) produced in the area, is easy to ingest even without water, and provides a malnutrition improving effect equal to or higher than that of conventional Ready-to-Use Therapeutic Foods. In addition, the food of the present invention can provide an effect equal to or higher than that provided when it contains an animal-derived material such as milk. Therefore, the food of the present invention is highly useful for the nutritional treatment and nutritional support of malnourished patients in developing regions.

This application is based on French Patent Application No. 1661851 (filing date: December 2, 2016), the contents of which are incorporated in full herein.

## Claims

1. A food comprising at least one grain selected from the group consisting of soybean, maize and sorghum, or a pulverized form of said at least one grain, lysine or a salt thereof, glutamine or a salt thereof, and methionine or a salt thereof, wherein
a content of the lysine or a salt thereof is 0.01 - 3 wt% relative to the food,
a content of the glutamine or a salt thereof is 0.01 - 3 wt% relative to the food, and
a content of the methionine or a salt thereof is 0.001 - 3 wt% relative to the food.

2. The food according to claim 1, wherein a content of said grain is 20 - 60 wt% relative to the food,
the content of the lysine or a salt thereof is 0.1 - 0.5 wt% relative to the food,
the content of the glutamine or a salt thereof is 0.1 - 0.8 wt% relative to the food, and
the content of the methionine or a salt thereof is 0.01 - 0.3 wt% relative to the food.

3. The food according to claim 1 or 2, wherein said grain includes soybean, maize and sorghum.

4. The food according to claim 3, wherein
a content of the soybean is 15 - 50 wt% relative to the food,
a content of the maize is 1 - 10 wt% relative to the food, and
a content of the sorghum is 0.5 - 5 wt% relative to the food.

5. The food according to any one of claims 1 to 4, further comprising at least one selected from proline or a salt thereof, and glycine or a salt thereof.

6. The food according to any one of claims 1 to 5, further comprising oil.

7. The food according to any one of claims 1 to 6, which does not contain milk or contains milk at not more than 20 wt% relative to the food.

8. The food according to any one of claims 1 to 7, which is substantially free of peanut.

9. The food according to any one of claims 1 to 8 for the medical treatment or prophylaxis of malnutrition.

10. The food according to claim 9, wherein the malnutrition is severe acute malnutrition.

11. The food according to any one of claims 1 to 10, which is a Ready-to-Use Therapeutic Food.

12. The non-medical use of the food according to any one of claims 1 to 11 for the prophylaxis or improvement of malnutrition.

13. The use according to claim 12, wherein the malnutrition is severe acute malnutrition.

14. A method for preparing a food according to any one of claims 1 to 11, which comprises the following steps:
(i) providing a material containing at least one grain selected from the group consisting of soybean, maize and sorghum, or a pulverized form of said at least one grain,
(ii) adding lysine or a salt thereof, glutamine or a salt thereof, and methionine or a salt thereof, to the material of step (i),
wherein a content of the lysine or a salt thereof is 0.01 - 3 wt% relative to the food,
a content of the glutamine or a salt thereof is 0.01 - 3 wt% relative to the food, and
a content of the methionine or a salt thereof is 0.001 - 3 wt% relative to the food.
